# EUROPÄISCHE PATENTSCHRIFT

(11) **EP 0 843 574 B1**
(45) Veröffentlichungstag und Bekanntmachung des Hinweises auf die Patenterteilung: **29.12.1999**
(21) Anmeldenummer: 95932017.7
(22) Anmeldetag: 08.09.1995
(51) Int. Cl.: A61N 1/05

(54) **CUFF-ELEKTRODE**
CUFF ELECTRODE
BRASSARD A ELECTRODES

(30) Priorität: 16.09.1994 DE 4433111
(43) Veröffentlichungstag der Anmeldung: 27.05.1998
(73) Patentinhaber: Fraunhofer-Gesellschaft zur Förderung der angewandten Forschung e.V., 80636 München (DE)
(72) Erfinder: STIEGLITZ, Thomas, D-66954 Pirmasens (DE); MEYER, Jörg-Uwe, D-66386 St. Ingbert (DE)
(86) Internationale Anmeldenummer: EP9503545
(87) Internationale Veröffentlichungsnummer: WO9608290

(56) Entgegenhaltungen:
- EP-A- 0 408 358
- WO-A-87/07825
- WO-A-91/17791
- WO-A-93/20887
- US-A- 3 654 933
- US-A- 3 738 368
- US-A- 3 774 618
- US-A- 3 955 560

## Beschreibung

Die Erfindung betrifft eine Cuff-Elektrode gemäß dem Oberbegriff des Anspruchs 1, wie sie z.B. aus WO 91/17791 bekannt ist.

Weiter wird verwiesen auf den Artikel "Selective control of muscle activation with a multipolar nerve cuff-electrode" in IEEE Transactions on biomedical engineering, Vol. 40, No. 7, July 1993, sowie auf die US-Patente 4,750,499, 5,038,781 und 5,324,322.

### Technisches Anwendungsgebiet

Das technische Anwendungsgebiet der neuen **FL**exiblen **I**nterdigitalen **C**uff-Elektrode (FLIC) ist in der Neuroprothetik zu finden. Hier werden neue Möglichkeiten gesucht, Nervenbündel mit einer Vielzahl von Elektroden schonend und langfristig zu kontaktieren. Durch den Einsatz von technischen Hilfssystemen im menschlichen Körper sollte es durch die Registrierung von Nervensignalen und durch die Stimulation möglich sein, ausgefallene Körperfunktionen wieder herzustellen bzw. nachzuahmen.

### Stand der Technik; Nachteile des Standes der Technik

### Starre Manschettenelektroden

Extraneurale, manschettenartige Elektroden, sogenannte Cuff-Elektroden, die zirkulär um den Nerv gelegt werden, gehören seit Ende der 70^{er} Jahre zu den erfolgreichsten biomedizinischen Elektroden auf dem Markt. Für starre Bauformen, sogenannte "Split Cylindre" Cuff-Elektroden bestehen einige US Patente: 3,774,618, 3,738,368, 3,654,933. Einige implantierte Elektroden sind seit 15 Jahren sicher im Einsatz. Ihre Nachteile bestanden und bestehen in ihrer geringen Selektivität aufgrund ihrer geringen Anzahl von Elektroden (zirkular maximal 4). Die Anordnung von drei Elektroden in Längsrichtung der Manschette und der Einsatz von transversalen Steuerströmen ermöglicht eine Verbesserung der Selektivität und der Reizung tiefer gelegener Anteile des Nerven. Bedingt durch ihre relativ starre Form kann es bei postoperativen oedematösen Schwellungen zur Schädigung der Nerven durch den entstehenden Druck der Elektrode auf den Nerven führen. Wird der Durchmesser der Manschette größer gewählt, verschlechtern sich durch einwachsendes Bindegewebe die Ableit- und Stimulationsbedingungen beträchtlich.

### Flexible Manschettenelektroden

Mit der Einführung der sogenannten Spiral Cuff-Elektroden wurde ein Weg zur Verringerung eines postoperativen Druckanstiges gegangen. Eine vorgedehnte Silikonfolie wird bei dieser Bauform mit einer ungedehnten Folie mit medizinischem Silikonkleber verbunden. Die Elektroden bestehen aus Platindrähten, die zwischen die Folien gelegt werden. Die aktive Elektrodenfläche wird durch Öffnen von Fenstern (Skalpell, Stanzeisen) in der vorgedehnten Folie erstellt.

Aufgrund der Zugspannung der vorgedehnten Folie rollt sich die Elektrode spiralförmig auf. Bei einer Anschwellung des Nerven kann sich die spiralförmige Manschettenelektrode etwas aufdehnen und dadurch einen Druckanstieg teilweise ausgleichen.

### Helix Elektroden

Mit dem Begriff der Helix-Elektrode werden schraubenförmig um den Nerv gewundene Elektroden bezeichnet- Sie sind in mono- und bipolarer Form mit maximal zwei Elektroden im Einsatz. Durch die offene Struktur der Elektrode ist die Gefahr einer Druckschädigung des Nerven durch Schwellungen gering, eine Feldbegrenzung auf das Innere der Elektrode bei der Stimulation (geringerer Energiebedarf) ist jedoch nicht möglich. Sie besitzt keine Möglichkeit, bestimmte Teile des Nervenquerschnittes selektiv zu reizen.

Aufgabe der Erfindung ist es, die Cuff-Elektrode gemäß dem Oberbegriff des Anspruchs 1 zu verbessern. Insbesondere besteht die Aufgabe darin, den Auflagedruck der Elektrode auf die Nerven zu verringern. Eine bidirektionale Kopplung an die Nerven mit hoher räumlicher und zeitlicher Auflösung soll eine Ableitung von Nervensignalen und/oder eine elektrische Stimulation von Nervenfasern ermöglichen.

Dies wird erfindungsgemäß durch Anspruch 1 gelöst. Vorteilhafte Ausgestaltungen sind in den Unteransprüchen gekennzeichnet.

### Mit der Erfindung gelöste Aufgabe

Durch den Einsatz erhabener Elektroden wird die effektive Berührungsfläche vermindert, so daß der "Negativabdruck" einer Punktstruktur entsteht, der zu einer besseren Biokompatibilität und zu einer besseren Fixierung durch Fibroblasten führen kann. Die neue Cuff-Struktur erlaubt eine Feldbegrenzung auf das Elektrodeninnere, wodurch die benötigte Energiemenge zur Stimulation niedrig gehalten werden kann. Die hohe Anzahl der Elektroden erlaubt eine hohe räumliche und zeitliche Auflösung und den Einsatz von tripolaren Anordnungen zur Konzentration des elektrischen Feldes sowie den Einsatz von transversalen Steuerströmen zur Erregung tiefer Nervenstrukturen. Siehe hierzu die vorne zitierte Zeitschrift IEEE Transactions 1993.

Mit der Fingerstruktur werden zwei Ziele verfolgt: Zum einen wird bei einer oedematösen Schwellung der Druck der Elektrode auf den Nerven verringert, da die einzelnen Streifen bzw. Finger sich einer nicht gleichmäßigen Schwellung besser anpassen können. Im Gegensatz zu konventionellen Cuff-Elektroden wird den physiologischen Unregelmäßigkeiten des Nerven Rechnung getragen. Zum anderen ermöglicht die Struktur eine Diffusion von Stoffwechselprodukten durch die FingerZwischenräume, die sich in der postoperativen Regenerationsphase positv auswirken kann, da schädliche Stoffwechselendprodukte abtransportiert werden können. Dies wird ebenfalls durch die erhabenen Elektroden und die damit verbundenen Zwischenräume zwischen den Elektroden erreicht.

Der Einsatz der flexiblen interdigitalen Cuff-Elektrode (FLIC) ist an peripheren Nerven und im Rückenmark geplant. Eine epidurale und subdurale Implantation um die Wurzeln des Rückenmarkes ist denkbar. Bei einer Implantation um das Rückenmark herum, können nach einer Laminektomie zwei FLIC um das Rückenmark radial zwischen zwei Wurzeln gelegt werden.

### Erzeugte Verbesserung und Vorteile gegenüber dem Stand der Technik

- hohe zeitlich-räumliche Auflösung
- Anpassung des Radius der Elektrode an Unregelmäßigkeiten entlang des Nerven aufgrund der Interdigitaistruktur möglich
- Verringerung des Druckes auf den Nerven bei einer lokalen postoperativen oedematösen Schwellung aufgrund der Implantation durch die Interdigitalstruktur
- Verbesserung der Biokompatibilität durch erhabene Elektroden → Verringerung der effektiven Auflagefläche.

### Grundzüge der Erfindung

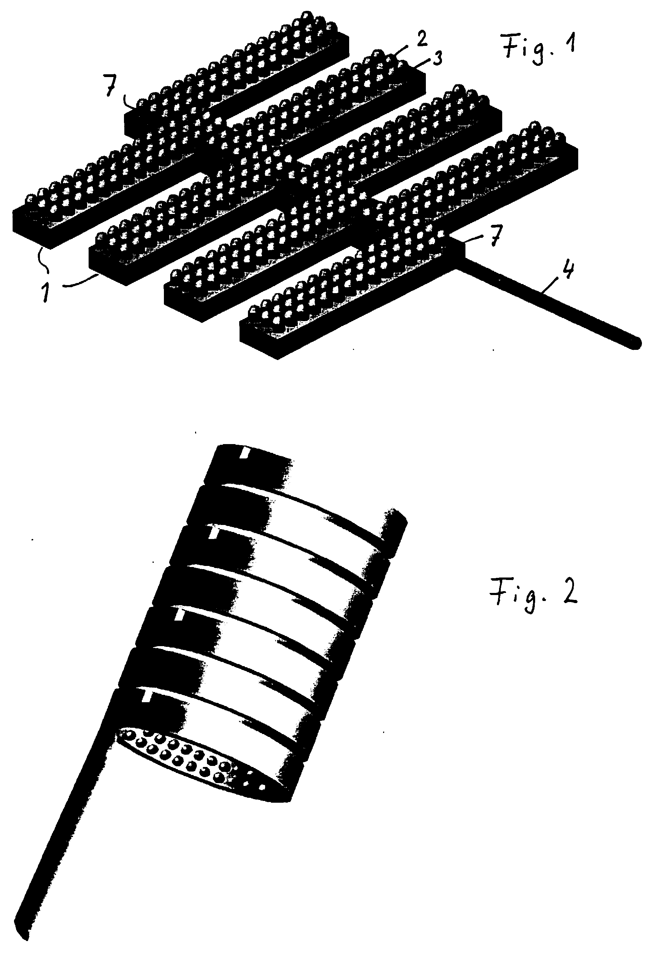

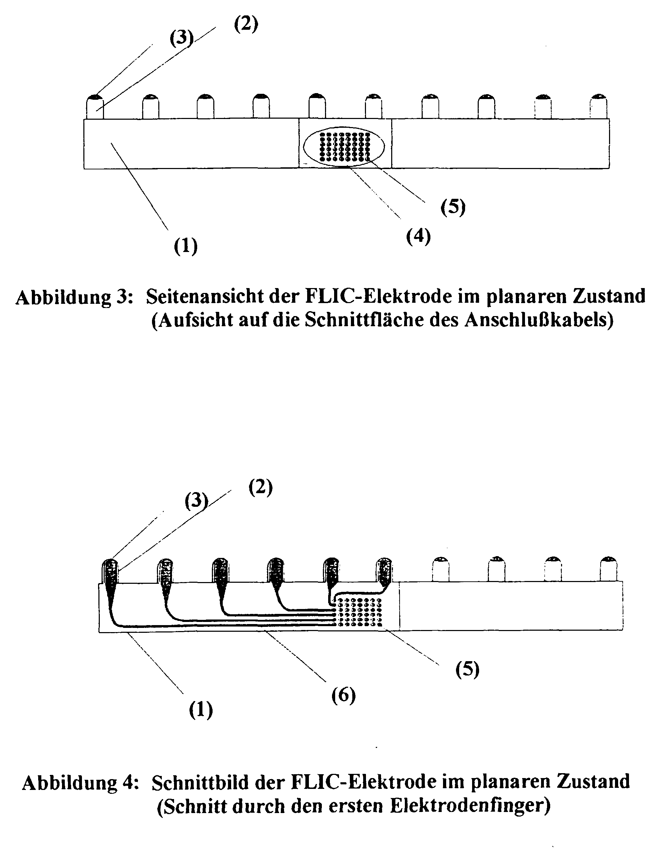

Die Erfindung ist in perspektivischer Darstellung in den Figuren 1 und 2 schematisch und in Fig. 3 und 4 im Schnitt dargestellt. Figur 1 zeigt die flexible interdigitale Cuff-Elektrode in planarem Zustand und Figur 2 die Cuff-Elektrode in aufgerollter Form, wobei der Nerv im Inneren der aufgerollten Finger zu liegen kommt. In der Figur ist der Nerv natürlich nicht dargestellt. Abb. 3 zeigt die Seitenansicht der FLIC-Elektrode im planaren Zustand (Aufsicht auf die Schnittfläche des Anschlußkabels), Abb. 4 das Schnittbild der FLIC-Elektrode im planaren Zustand (Schnitt durch den ersten Elektrodenfinger).

Die FLIC besteht aus einem flexiblen, mehrschichtigen Substrat in Fingerstruktur 1 aus nichtleitendem Silicon, auf dem sich erhabene Elektroden 3 aus leitfähigem Silikon befinden. Die zu den Elektroden 3 führenden Leiterbahnen 5 verlaufen zwischen den nicht leitfähigen Schichten der Finger und bestehen ebenfalls aus leitfähigem Silikon. Diese Leiterbahnen sind in den Figuren 3 und 4 nur für 48 Elektroden dargestellt, da bei den dargestellten 45 Elektroden pro Finger nach Fig. 1 dies zeichnerisch schlecht darstellbar ist. Die Cuff-Elektrode nach Fig. 3 und 4 hat 6 Elektroden pro Finger mit 4 Fingern links und 4 Fingern auf der rechten Seite. Die Leiterbahnen und Elektroden werden durch Mikrostrukturabformung mit Negativformen, die in konventioneller Siliziumtechnologie entstehen, erstellt, z.B. werden Formen vorgesehen, in die Siliconkautschuk gerakelt wird (gelegt), der anschließend thermisch vernetzt wird. Als Formen können sowohl naß-chemisch geätzte Strukturen in Silicium dienen, siehe z.B. Anton Heuberger Mikromechanik "Mikrofertigung mit Methoden der Halbleitertechnologie", Springer-Verlag Berlin, Heidelberg, New York, London, Paris, Tokyo 1989, als auch mittels Präzisionsmechanik hergestellte Formen.

Durch die Einstellung verschiedener Vorspannungen im Mehrschichtaufbau oder durch den Einsatz von Formgedächtnismetallen (Shape Memory Alloy) oder durch elektrochemische Aktoren entsteht die aufgerollte Form der FLIC. Die Formgedächtnismetalle werden in Form einzelner Streifen innerhalb der isolierenden Materialschichten eingefügt. Da die Vorspannung relativ gering zu sein braucht, kann eine Lage aus Formgedächtnismaterial genügen. Das Formgedächtnismetall ist von allen Seiten mit isolierendem Silikonkautschuk 1 umgeben und besitzt keinen Kontakt zu den leitfähigen Strukturen der Elektroden 3 und Zuleitungen 5, 6. Die Elektroden mit den Leitungen in den Fingern und dem Zuleitungskabel 4 sind gegeneinander isoliert und sind an eine nicht dargestellte Verarbeitungseinheit zum Beaufschlagen der Elektroden mit Reizströmen verbunden. Die Leitungen 5, 6 können dabei derart verschaltet sein, daß auch Ableitungen von Nervensignalen in der Verarbeitungseinheit empfangen werden können.

Die Leiterbahnen und Elektroden werden bevorzugt aus leitfähigen Silikonkautschuk in Mikrostrukturtechnik hergestellt, ebenso die isolierenden dazwischenliegenden Silikonschichten. Die Verwendung von Silikonfolien ist nicht vorgesehen. (3) ist die aktive Elektrodenstruktur aus leitendem Silikonkautschuk. Als Modifikation zur Verringerung der Elektrodenübergangsimpedanz (besseres Signal-Rausch-Verhältnis bei der Ableitung) kann eine galvanische Nachbehandlung erfolgen, z.B. eine galvanische Abscheidung von Kohlenstoff, Gold, Platin oder Iridium auf der in Abb. 3 gezeichneten "Kuppe". Bei der Abscheidung von Platin bzw. Iridium erhöht sich zudem die Ladungsmenge, die pro Stimulationspuls pro Elektrode mittels reversibler elektrochemischer Prozesse eingebracht werden kann.

Abb. 3 und 4 zeigen die erhabene Elektrodenstruktur mit einem Grundmaterial aus nichtleitendem Silikonkautschuk 2. Der nichtleitende Silikonkautschuk 2 liegt quasi wie eine Hülse um den leitenden Kern und ermöglicht eine elektrische Isolation der einzelnen Elektroden 3 gegeneinander (s. Abb. 4).

Die rechte Seite von Abb. 4 ist nicht angeschnitten, da dort bereits der zweite Elektrodenfinger zu sehen ist, der gegenüber dem ersten nach hinten versetzt ist.

Der nichtleitende Silikonkautschuk gibt die Trägerstruktur für die Zuleitungen 6 und unterstützt diese mechanisch und isoliert die einzelnen Zuleitungen 5, 6 auch in dem Anschlußkabel 4.

Die erhabenen Elektroden sind nach den Figuren 1 - 4 stäbchenförmig mit einer haibkugelförmigen Elektrodenspitze ausgebildet. Sie können natürlich auch Stäbchen mit quadratischem oder mehreckigem Querschnitt sein und ebenfalls einer abgerundeten Spitze. Diese Ausbildung bewirkt eine erhebliche Verringerung der effektiven Berührfläche der Cuff-Elektrode mit den Nerven. Auf dem Nervengewebe ergibt dies dann ein punktförmiges Raster, wobei die Punkte auf Grund des Eindrucks in dem Gewebe eine flächige Form haben. Graphisch gesehen, bilden die Hohlräume zwischen den erhabenen Strukturen die Form eines Gitters oder Netzes. Durch die Fingerstruktur können sich die einzelnen Finger um den Nerven herumlegen mit einem an den Nerven angepaßten u.U. unterschiedlichen mittleren Durchmesser.

Die Zahl der Elektroden kann dabei nach Art der Anwendung schwanken. Bevorzugt sind in jedem Fall mehrere Elektroden pro Finger, z.B. 4 - 50 Elektroden pro Finger. Auch die Zahl der Finger richtet sich nach Art der Anwendung und der Dicke des Nervs. Vorgesehen sind z.B. mindestens zwei Finger pro Seite, d.h. im Ganzen vier Finger, vorzugsweise 4 - 5 oder mehr Finger pro Seite.

Nach Figur 1 und 2 sind die Finger links und rechts von einem zentralen Teil 7 angeordnet mit je 4 Fingern. Selbstverständlich sind auch andere Anordnungen der Finger um einen zentralen Teil denkbar. So können z.B. alle Finger wie bei einer Hand nur auf einer Seite angeordnet sein und sich dann wie eine Hand um den Nerven legen. Oder es können auch z.B. jeweils 2 Finger nebeneinander liegen und auf der anderen Seite ein entsprechender Leerraum. Im einfachsten Fall kann es auch genügen, wenn auf einer Seite nur 1 Finger ist und auf der anderen Seite 2 Finger mit dem Zwischenraum für den einen Finger analog zur Figur 1, oder wie oben angedeutet die 3 Finger entlang eines zentralen Teils 7 auf der gleichen Seite.

### Ausführungsbeispiel

Entlang der Längsachse der Flexiblen Interdigitalen Cuff-Elektrode befinden sich auf jeder Seite eines Mittelsteges 4 bis 6 "Finger" mit jeweils 6 - 45 erhabenen Elektroden, die beliebig konfiguriert werden können, um die gleiche FLIC prinzipiell sowohl für die Ableitung von Elektroneurogrammen (ENG) als auch für die Stimulation einsetzen zu können. Die möglichen Maße der FLIC betragen ca. 10 mm Länge bei ca. 1,4 mm - 40 mm Durchmesser.

## Patentansprüche

1. Cuff-Elektrode, die als extraneurale, manschettenförmige Elektrode um ein biologisches Gewebe, z.B. einem Nerv, anlegbar ist, wobei erhabene Elektroden (3) auf einer Folie aus nichtleitenden Materialschichten (1) vorgesehen sind und die einzelnen Elektroden (3) durch Leiterbahnen (5, 6), die zwischen den nichtleitenden Schichten angeordnet sind, kontaktiert sind und zur Gewährleistung der manschettenförmigen Struktur mindestens eine der Schichten aus Formgedächtnismaterial besteht oder unter mechanischer Vorspannung steht,
**dadurch gekennzeichnet**,
daß die Materialschichten mit den Elektroden (3) fingerförmig ausgebildet sind, derart, daß sie im den Nerv umgreifenden Zustand eng aneinander liegen und eine Vielzahl von stiftförmigen Elektroden in einer Array-Anordnung vorgesehen sind, so daß die Auflagekräfte auf die Nerven minimiert sind.

2. Cuff-Elektrode nach Anspruch 1,
**dadurch gekennzeichnet**,
daß 4 - 50 Elektroden, vorzugsweise 6 - 20, pro Finger vorgesehen sind und stäbchenförmig mit rundem oder mehreckigem Querschnitt und einer kalottenförmigen Elektrodenspitze ausgebildet sind und aus leitfähigem Silikon bestehen.

3. Cuff-Elektrode nach Anspruch 1,
**dadurch gekennzeichnet**,
daß die Elektroden (3), Leiterbahnen (5, 6) und Zuleitungskabel (4) mittels Mikrostrukturabformung mit Negativformen hergestellt sind.

4. Cuff-Elektrode nach Anspruch 1,
**dadurch gekennzeichnet**,
daß die Elektroden derart angeordnet sind, daß eine hohe zeitlich-räumliche Auflösung sowohl für die Stimulation von Nerven als auch für die Ableitung von Nervensignalen erreicht wird, so daß z.B. tripolare Elektrodenverschaltungen mit transversalen Steuerströmen ausbildbar sind.

5. Cuff-Elektrode nach einem der Ansprüche 1 - 4,
**dadurch gekennzeichnet**,
daß die Elektrodenspitzen bzw. Berührungsflächen mit Platin, Iridium oder Iridiumoxid beschichtet sind.

6. Cuff-Elektrode nach Anspruch 1,
**dadurch gekennzeichnet**,
daß das Formgedächtnismaterial aus Metall oder Kunststoff ist und die mechanische Vorspannung z.B. durch einen Federstahl oder federnden Kunststoff bewirkt ist.

7. Cuff-Elektrode nach Anspruch 1,
**dadurch gekennzeichnet**,
daß mindestens 2 Finger (1) pro Seite, vorzugsweise 3 - 5 Finger, vorgesehen sind, und die Finger (1) derart links und rechts von einem zentralen Teil (7) angeordnet sind, daß sie im den Nerv umgreifenden Zustand ineinander greifen.

## Claims

1. Cuff electrode which may be applied as an extraneural, cuff-shaped electrode around a biological tissue, e.g. a nerve, raised electrodes (3) being provided on a foil made of non-conductive material layers (1) and contact to the individual electrodes (3) being supplied through track conductors (5,6) which are disposed between the non-conductive layers, and, in order to guarantee the cuff-shaped structure, at least one of the layers consisting of a shape memory material or being under initial mechanical stress,
**characterised in that**
the material layers with the electrodes (3) are configured finger-shaped, in such a way that they abut closely against one another when gripping round the nerve, and a plurality of pin-shaped electrodes is provided in an array arrangement, such that the bearing forces on the nerves are minimised.

2. Cuff electrode according to claim 1,
**characterised in that**
4 to 50, preferably 6 to 20, electrodes are provided per finger and are configured rod-like with round or polygonal cross-section and a dome-shaped electrode tip, and consist of conductive silicone.

3. Cuff electrode according to claim 1,
**characterised in that**
the electrodes (3), track conductors (5,6) and feed cables (4) are produced by means of microstructure moulding with negative moulds.

4. Cuff electrode according to claim 1,
**characterised in that**
the electrodes are disposed in such a way that a high time-space resolution is achieved both for the stimulation of nerves and also for carrying away nerve signals, such that e.g. tripolar electrode circuits with transverse control currents may be formed.

5. Cuff electrode according to one of claims 1 to 4,
**characterised in that**
the electrode tips or contact surfaces are coated with platinum, iridium or iridium oxide.

6. Cuff electrode according to claim 1,
**characterised in that**
the shape memory material is made of metal or plastics and the initial mechanical tension is caused by a spring steel or resilient plastics material.

7. Cuff electrode according to claim 1,
**characterised in that**
at least 2 fingers (1) per side, preferably 3 to 5 fingers, are provided, and the fingers (1) are arranged in such a way to the left and to the right of a central portion (7) that, when gripping round the nerve, they grip into one another.

## Revendications

1. Electrode à brassard qui peut être mise sous la forme d'une électrode extranerveuse, en forme de brassard, autour d'un tissu biologique, par exemple un nerf, des électrodes en saillie (3) étant prévues sur une feuille constituée de couches de matière non conductrices (1) et les différentes électrodes (3) étant mises en contact par des pistes conductrices (5, 6) disposées entre les couches non conductrices et, pour garantir la structure en forme de brassard ,l'une au moins des couches consistant en une matière conservant la mémoire des formes ou se trouvant sous une précontrainte mécanique,
caractérisée en ce que
les couches de matière sont constituées avec les électrodes (3) en forme de doigt, d'une manière telle qu'elles se trouvent dans une position qui entoure étroitement le nerf les unes à côté des autres, et qu'il est prévu un certain nombre d'électrodes en forme de broches selon une disposition en réseau, de telle sorte que les forces appuyant sur les nerfs soient réduites au minimum.

2. Electrode à brassard selon la revendication 1,
caractérisée en ce qu'
• on prévoit 4 à 50 électrodes, de préférence 6 à 20 , par doigt et,
• les électrodes sont constituées en forme de bâtonnet ayant une section transversale ronde ou polygonale et une pointe d'électrode en forme de calotte, en étant réalisées en silicone conducteur.

3. Electrode à brassard selon la revendication 1,
caractérisée en ce que
les électrodes (3), les pistes conductrices (5, 6) et le câble d'alimentation (4) sont réalisés par moulage d'une microstructure avec des moules négatifs.

4. Electrode à brassard selon la revendication 1,
caractérisée en ce que
les électrodes sont disposées de telle façon que l'on obtient une résolution élevée dans l'espace et dans le temps aussi bien pour la stimulation des nerfs que pour le prélèvement des signaux des nerfs, de telle sorte qu'on peut constituer par exemple des mises en circuit tripolaires d'électrodes avec des courants de commande transversaux.

5. Electrode à brassard selon la revendication 1 à 4,
caractérisée en ce que
les pointes des électrodes ou les surfaces de contact sont revêtues de platine, d'iridium ou d'oxyde d'iridium.

6. Electrode à brassard selon la revendication 1,
caractérisée en ce que
• la matière qui conserve la mémoire des formes est en métal ou en matière plastique, et
• la précontrainte mécanique est produite par exemple par un acier à ressort ou une matière plastique élastique.

7. Electrode à brassard selon la revendication 1,
caractérisée en ce qu'
on prévoit au moins deux doigts (1) par côté, de préférence 3 à 5 doigts, et les doigts (1) sont disposés à gauche et à droite d'une partie centrale (7), de telle sorte qu'ils viennent en prise les uns dans les autres dans une position qui entoure le nerf.
